# EUROPEAN PATENT APPLICATION

(11) **EP 2 495 313 A1**
(43) Date of publication of application: **05.09.2012**
(21) Application number: 10826671.9
(22) Date of filing: 25.10.2010
(51) Int. Cl.: C12N 5/10, A01H 5/00, A01H 5/08, A01H 5/10, C12N 15/09

(54) **PRODUCTION METHOD FOR GENETICALLY MODIFIED PLANT CELLS**

(30) Priority: 26.10.2009 JP 2009245416
(71) Applicant: National Institute Of Agrobiological Sciences, Ibaraki 305-8602 (JP)
(72) Inventor: TOKI, Seiichi, Tsukuba-shi Ibaraki 305-8602 (JP); OSAKABE, Keishi, Tsukuba-shi Ibaraki 305-8602 (JP)
(74) Representative: Cohausz & Florack
(86) International application number: PCT/JP2010/068858
(87) International publication number: WO 2011/052539

(57) **Abstract**

It has been found that the use of a plant cell in which a function of a protein involved in repair by nonhomologous end joining is artificially suppressed dramatically increases the efficiency of introductions of non-silent mutations in a repairing process by nonhomologous end joining which occurs after induction of a DNA double-strand break with a zinc finger nuclease.

SEQUENCE LISTING

<110>  National Institute of Agrobiological Sciences
<120>  Methods of generating genetically modified plant cells
<130>  dokugai128
<150>  JP 09/245416
<151>  2009-10-26
<160>  25
<170>  PatentIn version 3.1
<210>  1
<211>  621
<212>  PRT
<213>  Arabidopsis thaliana
<400>  1
Met Glu Leu Asp Pro Asp Asp Val Phe Arg Asp Glu Asp Glu Asp Pro
1               5                   10                  15
Glu Asn Asp Phe Phe Gln Glu Lys Glu Ala Ser Lys Glu Phe Val Val
            20                  25                  30
Tyr Leu Ile Asp Ala Ser Pro Lys Met Phe Cys Ser Thr Cys Pro Ser
        35                  40                  45
Glu Glu Glu Asp Lys Gln Glu Ser His Phe His Ile Ala Val Ser Cys
    50                  55                  60
Ile Ala Gln Ser Leu Lys Ala His Ile Ile Asn Arg Ser Asn Asp Glu
65                  70                  75                  80
Ile Ala Ile Cys Phe Phe Asn Thr Arg Glu Lys Lys Asn Leu Gln Asp
                85                  90                  95
Leu Asn Gly Val Tyr Val Phe Asn Val Pro Glu Arg Asp Ser Ile Asp
            100                 105                 110
Arg Pro Thr Ala Arg Leu Ile Lys Glu Phe Asp Leu Ile Glu Glu Ser
        115                 120                 125
Phe Asp Lys Glu Ile Gly Ser Gln Thr Gly Ile Val Ser Asp Ser Arg
    130                 135                 140
Glu Asn Ser Leu Tyr Ser Ala Leu Trp Val Ala Gln Ala Leu Leu Arg
145                 150                 155                 160
Lys Gly Ser Leu Lys Thr Ala Asp Lys Arg Met Phe Leu Phe Thr Asn
                165                 170                 175
Glu Asp Asp Pro Phe Gly Ser Met Arg Ile Ser Val Lys Glu Asp Met
            180                 185                 190
Thr Arg Thr Thr Leu Gln Arg Ala Lys Asp Ala Gln Asp Leu Gly Ile
        195                 200                 205
Ser Ile Glu Leu Leu Pro Leu Ser Gln Pro Asp Lys Gln Phe Asn Ile
    210                 215                 220
Thr Leu Phe Tyr Lys Asp Leu Ile Gly Leu Asn Ser Asp Glu Leu Thr
225                 230                 235                 240
Glu Phe Met Pro Ser Val Gly Gln Lys Leu Glu Asp Met Lys Asp Gln
                245                 250                 255
Leu Lys Lys Arg Val Leu Ala Lys Arg Ile Ala Lys Arg Ile Thr Phe
            260                 265                 270
Val Ile Cys Asp Gly Leu Ser Ile Glu Leu Asn Gly Tyr Ala Leu Leu
        275                 280                 285
Arg Pro Ala Ile Pro Gly Ser Ile Thr Trp Leu Asp Ser Thr Thr Asn
    290                 295                 300
Leu Pro Val Lys Val Glu Arg Ser Tyr Ile Cys Thr Asp Thr Gly Ala
305                 310                 315                 320
Ile Met Gln Asp Pro Ile Gln Arg Ile Gln Pro Tyr Lys Asn Gln Asn
                325                 330                 335
Ile Met Phe Thr Val Glu Glu Leu Ser Gln Val Lys Arg Ile Ser Thr
            340                 345                 350
Gly His Leu Arg Leu Leu Gly Phe Lys Pro Leu Ser Cys Leu Lys Asp
        355                 360                 365
Tyr His Asn Leu Lys Pro Ser Thr Phe Leu Tyr Pro Ser Asp Lys Glu
    370                 375                 380
Val Ile Gly Ser Thr Arg Ala Phe Ile Ala Leu His Arg Ser Met Ile
385                 390                 395                 400
Gln Leu Glu Arg Phe Ala Val Ala Phe Tyr Gly Gly Thr Thr Pro Pro

                    405                         410                         415
Arg Leu Val Ala Leu Val Ala Gln Asp Glu Ile Glu Ser Asp Gly Gly
                420                         425                         430
Gln Val Glu Pro Pro Gly Ile Asn Met Ile Tyr Leu Pro Tyr Ala Asn
            435                         440                         445
Asp Ile Arg Asp Ile Asp Glu Leu His Ser Lys Pro Gly Val Ala Ala
    450                         455                         460
Pro Arg Ala Ser Asp Asp Gln Leu Lys Lys Ala Ser Ala Leu Met Arg
465                         470                         475                         480
Arg Leu Glu Leu Lys Asp Phe Ser Val Cys Gln Phe Ala Asn Pro Ala
                        485                         490                         495
Leu Gln Arg His Tyr Ala Ile Leu Gln Ala Ile Ala Leu Asp Glu Asn
                500                         505                         510
Glu Leu Arg Glu Thr Arg Asp Glu Thr Leu Pro Asp Glu Glu Gly Met
            515                         520                         525
Asn Arg Pro Ala Val Val Lys Ala Ile Glu Gln Phe Lys Gln Ser Ile
    530                         535                         540
Tyr Gly Asp Asp Pro Asp Glu Glu Ser Asp Ser Gly Ala Lys Glu Lys
545                         550                         555                         560
Ser Lys Lys Arg Lys Ala Gly Asp Ala Asp Asp Gly Lys Tyr Asp Tyr
                        565                         570                         575
Ile Glu Leu Ala Lys Thr Gly Lys Leu Lys Asp Leu Thr Val Val Glu
                580                         585                         590
Leu Lys Thr Tyr Leu Thr Ala Asn Asn Leu Leu Val Ser Gly Lys Lys
            595                         600                         605
Glu Val Leu Ile Asn Arg Ile Leu Thr His Ile Gly Lys
    610                         615                         620


<210>  2
<211>  1866
<212>  DNA
<213>  Arabidopsis thaliana
<220>
<221>  CDS
<222>  (1)..(1863)
<223>
<400>  2
atg gaa ttg gac cca gat gat gtt ttc aga gat gaa gac gaa gac cca        48
Met Glu Leu Asp Pro Asp Asp Val Phe Arg Asp Glu Asp Glu Asp Pro
1               5                   10                  15
gaa aac gat ttc ttt cag gaa aaa gag gct tcc aaa gaa ttt gta gtc        96
Glu Asn Asp Phe Phe Gln Glu Lys Glu Ala Ser Lys Glu Phe Val Val
            20                  25                  30
tat cta att gat gct tct ccc aaa atg ttc tgc tct act tgc ccc tct       144
Tyr Leu Ile Asp Ala Ser Pro Lys Met Phe Cys Ser Thr Cys Pro Ser
        35                  40                  45
gag gaa gag gac aaa caa gaa agt cat ttt cac att gct gtt agt tgc       192
Glu Glu Glu Asp Lys Gln Glu Ser His Phe His Ile Ala Val Ser Cys
    50                  55                  60
att gct cag tca ctc aag gct cat att atc aat cgg tca aat gat gaa       240
Ile Ala Gln Ser Leu Lys Ala His Ile Ile Asn Arg Ser Asn Asp Glu
65                  70                  75                  80
ata gct ata tgt ttc ttt aac act cgt gaa aag aag aat ctt cag gat       288
Ile Ala Ile Cys Phe Phe Asn Thr Arg Glu Lys Lys Asn Leu Gln Asp
                85                  90                  95
cta aat ggt gtt tat gtc ttt aat gtt cca gag agg gat agt att gac       336
Leu Asn Gly Val Tyr Val Phe Asn Val Pro Glu Arg Asp Ser Ile Asp
            100                 105                 110
cgt cca aca gcg agg ctg att aaa gaa ttc gat ctc ata gaa gaa tca       384
Arg Pro Thr Ala Arg Leu Ile Lys Glu Phe Asp Leu Ile Glu Glu Ser
        115                 120                 125
ttt gat aag gaa att ggc agt caa act ggt att gtc tca gat tct cgg       432
Phe Asp Lys Glu Ile Gly Ser Gln Thr Gly Ile Val Ser Asp Ser Arg
    130                 135                 140

gag aat tcc ctt tat agt gct ctc tgg gtt gca caa gca cta ctg cgc 480
Glu Asn Ser Leu Tyr Ser Ala Leu Trp Val Ala Gln Ala Leu Leu Arg
145                 150                 155                 160
aaa gga tct tta aag aca gca gat aaa cgc atg ttt cta ttc act aat 528
Lys Gly Ser Leu Lys Thr Ala Asp Lys Arg Met Phe Leu Phe Thr Asn
                165                 170                 175
gaa gat gat cct ttt ggg agt atg agg atc tct gtt aaa gag gat atg 576
Glu Asp Asp Pro Phe Gly Ser Met Arg Ile Ser Val Lys Glu Asp Met
            180                 185                 190
acg agg acg acg ttg cag aga gct aag gat gca caa gac ctt ggc atc 624
Thr Arg Thr Thr Leu Gln Arg Ala Lys Asp Ala Gln Asp Leu Gly Ile
        195                 200                 205
tca atc gag ctt cta cca ctc agt caa cct gat aag caa ttc aat ata 672
Ser Ile Glu Leu Leu Pro Leu Ser Gln Pro Asp Lys Gln Phe Asn Ile
    210                 215                 220
act ctc ttc tac aag gat cta att gga tta aac agt gat gaa ctt acg 720
Thr Leu Phe Tyr Lys Asp Leu Ile Gly Leu Asn Ser Asp Glu Leu Thr
225                 230                 235                 240
gag ttc atg cca tca gtt gga cag aaa tta gag gac atg aag gac caa 768
Glu Phe Met Pro Ser Val Gly Gln Lys Leu Glu Asp Met Lys Asp Gln
                245                 250                 255
ctg aag aag cgt gta ctt gcc aag aga ata gca aaa aga att acg ttt 816
Leu Lys Lys Arg Val Leu Ala Lys Arg Ile Ala Lys Arg Ile Thr Phe
            260                 265                 270
gtg atc tgt gat ggc ttg tct att gaa ctg aat ggt tat gct tta ctc 864
Val Ile Cys Asp Gly Leu Ser Ile Glu Leu Asn Gly Tyr Ala Leu Leu
        275                 280                 285
cgt ccg gct att cct ggc agc ata acc tgg ctt gac tct aca act aat 912
Arg Pro Ala Ile Pro Gly Ser Ile Thr Trp Leu Asp Ser Thr Thr Asn
    290                 295                 300
ctt cct gtg aag gtg gaa aga tca tat atc tgt acg gac act gga gct 960
Leu Pro Val Lys Val Glu Arg Ser Tyr Ile Cys Thr Asp Thr Gly Ala
305                 310                 315                 320
ata atg caa gat cct ata caa cgg att cag cct tac aaa aat cag aat 1008
Ile Met Gln Asp Pro Ile Gln Arg Ile Gln Pro Tyr Lys Asn Gln Asn
                325                 330                 335
atc atg ttt act gtg gaa gag cta tcc caa gta aaa aga atc tca act 1056
Ile Met Phe Thr Val Glu Glu Leu Ser Gln Val Lys Arg Ile Ser Thr
            340                 345                 350
gga cac tta aga ctg ctg ggg ttc aag cca ctt agt tgc ctc aaa gat 1104
Gly His Leu Arg Leu Leu Gly Phe Lys Pro Leu Ser Cys Leu Lys Asp
        355                 360                 365
tat cac aat ttg aag cct tct aca ttt ctc tat cca agt gat aag gaa 1152
Tyr His Asn Leu Lys Pro Ser Thr Phe Leu Tyr Pro Ser Asp Lys Glu
    370                 375                 380
gtt ata ggg agc act cgt gca ttc att gcc ttg cat aga tcc atg ata 1200
Val Ile Gly Ser Thr Arg Ala Phe Ile Ala Leu His Arg Ser Met Ile
385                 390                 395                 400
cag ctt gaa cgt ttt gct gtt gca ttt tat ggc ggt act aca cct ccg 1248
Gln Leu Glu Arg Phe Ala Val Ala Phe Tyr Gly Gly Thr Thr Pro Pro
                405                 410                 415
aga ttg gtt gct ctt gtt gct cag gat gaa atc gag agt gat ggt ggt 1296
Arg Leu Val Ala Leu Val Ala Gln Asp Glu Ile Glu Ser Asp Gly Gly
            420                 425                 430
caa gtg gag cca cca ggg ata aac atg ata tat ctt cca tat gct aat 1344
Gln Val Glu Pro Pro Gly Ile Asn Met Ile Tyr Leu Pro Tyr Ala Asn
        435                 440                 445
gat att aga gat atc gac gag ttg cat tca aaa cca ggt gta gct gct 1392
Asp Ile Arg Asp Ile Asp Glu Leu His Ser Lys Pro Gly Val Ala Ala
    450                 455                 460
cct cgc gca tca gat gat cag ctt aaa aaa gca tca gct cta atg cga 1440
Pro Arg Ala Ser Asp Asp Gln Leu Lys Lys Ala Ser Ala Leu Met Arg
465                 470                 475                 480
cgt cta gag tta aaa gac ttt tcg gtt tgc caa ttt gct aat ccc gct 1488
Arg Leu Glu Leu Lys Asp Phe Ser Val Cys Gln Phe Ala Asn Pro Ala

485                 490                 495
ttg cag aga cac tat gct atc tta caa gcc att gca ctg gat gaa aac      1536
Leu Gln Arg His Tyr Ala Ile Leu Gln Ala Ile Ala Leu Asp Glu Asn
            500                 505                 510
gag ctt cgt gaa acc aga gat gaa aca ctt cct gac gaa gaa gga atg      1584
Glu Leu Arg Glu Thr Arg Asp Glu Thr Leu Pro Asp Glu Glu Gly Met
        515                 520                 525
aac aga cct gca gtt gta aaa gca ata gaa caa ttt aag cag tcg att      1632
Asn Arg Pro Ala Val Val Lys Ala Ile Glu Gln Phe Lys Gln Ser Ile
    530                 535                 540
tat ggc gat gac cct gat gaa gaa agt gac tca ggg gca aag gag aaa      1680
Tyr Gly Asp Asp Pro Asp Glu Glu Ser Asp Ser Gly Ala Lys Glu Lys
545                 550                 555                 560
tca aaa aag cga aaa gcc ggt gac gca gat gat gga aaa tat gac tat      1728
Ser Lys Lys Arg Lys Ala Gly Asp Ala Asp Asp Gly Lys Tyr Asp Tyr
                565                 570                 575
ata gag ctt gca aaa acc gga aag ctg aag gac ttg act gtg gtc gaa      1776
Ile Glu Leu Ala Lys Thr Gly Lys Leu Lys Asp Leu Thr Val Val Glu
            580                 585                 590
ctg aag aca tat ctg act gcc aac aat ctc ctc gtc agc ggg aag aaa      1824
Leu Lys Thr Tyr Leu Thr Ala Asn Asn Leu Leu Val Ser Gly Lys Lys
        595                 600                 605
gaa gtt ctg atc aat cgg att ctg act cac att ggt aaa taa              1866
Glu Val Leu Ile Asn Arg Ile Leu Thr His Ile Gly Lys
    610                 615                 620


<210>  3
<211>  680
<212>  PRT
<213>  Arabidopsis thaliana
<400>  3
Met Ala Arg Asn Arg Glu Gly Leu Val Leu Val Leu Asp Val Gly Pro
1               5                   10                  15
Ala Met Arg Ser Val Leu Pro Asp Val Glu Lys Ala Cys Ser Met Leu
            20                  25                  30
Leu Gln Lys Lys Leu Ile Tyr Asn Lys Tyr Asp Glu Val Gly Ile Val
        35                  40                  45
Val Phe Gly Thr Glu Glu Thr Gly Asn Glu Leu Ala Arg Glu Ile Gly
    50                  55                  60
Gly Tyr Glu Asn Val Thr Val Leu Arg Asn Ile Arg Val Val Asp Glu
65                  70                  75                  80
Leu Ala Ala Glu His Val Lys Gln Leu Pro Arg Gly Thr Val Ala Gly
                85                  90                  95
Asp Phe Leu Asp Ala Leu Ile Val Gly Met Asp Met Leu Ile Lys Met
            100                 105                 110
Tyr Gly Asn Ala His Lys Gly Lys Lys Arg Met Cys Leu Ile Thr Asn
        115                 120                 125
Ala Ala Cys Pro Thr Lys Asp Pro Phe Glu Gly Thr Lys Asp Asp Gln
    130                 135                 140
Val Ser Thr Ile Ala Met Lys Met Ala Ala Glu Gly Ile Lys Met Glu
145                 150                 155                 160
Ser Ile Val Met Arg Ser Asn Leu Ser Gly Asp Ala His Glu Arg Val
                165                 170                 175
Ile Glu Glu Asn Asp His Leu Leu Thr Leu Phe Ser Ser Asn Ala Ile
            180                 185                 190
Ala Lys Thr Val Asn Val Asp Ser Pro Leu Ser Leu Leu Gly Ser Leu
        195                 200                 205
Lys Thr Arg Arg Val Ala Pro Val Thr Leu Phe Arg Gly Asp Leu Glu
    210                 215                 220
Ile Asn Pro Thr Met Lys Ile Lys Val Trp Val Tyr Lys Lys Val Ala
225                 230                 235                 240
Glu Glu Arg Leu Pro Thr Leu Lys Met Tyr Ser Asp Lys Ala Pro Pro
                245                 250                 255
Thr Asp Lys Phe Ala Lys His Glu Val Lys Val Asp Tyr Asp Tyr Lys

                260                 265                 270
Val Thr Ala Glu Ser Thr Glu Val Ile Ala Pro Glu Glu Arg Ile Lys
        275                 280                 285
Gly Phe Arg Tyr Gly Pro Gln Val Ile Pro Ile Ser Pro Asp Gln Ile
    290                 295                 300
Glu Thr Leu Lys Phe Lys Thr Asp Lys Gly Met Lys Leu Leu Gly Phe
305                 310                 315                 320
Thr Glu Ala Ser Asn Ile Leu Arg His Tyr Tyr Met Lys Asp Val Asn
                325                 330                 335
Ile Val Val Pro Asp Pro Ser Lys Glu Lys Ser Val Leu Ala Val Ser
            340                 345                 350
Ala Ile Ala Arg Glu Met Lys Glu Thr Asn Lys Val Ala Ile Val Arg
        355                 360                 365
Cys Val Trp Arg Asn Gly Gln Gly Asn Val Val Val Gly Val Leu Thr
    370                 375                 380
Pro Asn Val Ser Glu Arg Asp Asp Thr Pro Asp Ser Phe Tyr Phe Asn
385                 390                 395                 400
Val Leu Pro Phe Ala Glu Asp Val Arg Glu Phe Pro Phe Pro Ser Phe
                405                 410                 415
Asn Lys Leu Pro Ser Ser Trp Lys Pro Asp Glu Gln Gln Gln Ala Val
            420                 425                 430
Ala Asp Asn Leu Val Lys Met Leu Asp Leu Ala Pro Ser Ala Glu Glu
        435                 440                 445
Glu Val Leu Lys Pro Asp Leu Thr Pro Asn Pro Val Leu Gln Arg Phe
    450                 455                 460
Tyr Glu Tyr Leu Glu Leu Lys Ser Lys Ser Thr Asp Ala Thr Leu Pro
465                 470                 475                 480
Pro Met Asp Gly Thr Phe Lys Arg Leu Met Glu Gln Asp Pro Glu Leu
                485                 490                 495
Ser Ser Asn Asn Lys Ser Ile Met Asp Thr Phe Arg Gly Ser Phe Glu
            500                 505                 510
Val Lys Glu Asn Pro Lys Leu Lys Lys Ala Ser Lys Arg Leu Leu Arg
        515                 520                 525
Asp Lys Pro Ser Gly Ser Asp Asp Glu Asp Asn Arg Met Ile Thr Tyr
    530                 535                 540
Asp Ala Lys Glu Asn Lys Ile Asp Ile Val Gly Asp Ala Asn Pro Ile
545                 550                 555                 560
Gln Asp Phe Glu Ala Met Ile Ser Arg Arg Asp Lys Thr Asp Trp Thr
                565                 570                 575
Glu Lys Ala Ile Thr Gln Met Lys Asn Leu Ile Met Lys Leu Val Glu
            580                 585                 590
Asn Cys Thr Asp Glu Gly Asp Lys Ala Leu Glu Cys Val Leu Ala Leu
        595                 600                 605
Arg Lys Gly Cys Val Leu Glu Gln Glu Pro Lys Gln Phe Asn Glu Phe
    610                 615                 620
Leu Asn His Leu Phe Lys Leu Cys Gln Glu Arg Asn Leu Ser His Leu
625                 630                 635                 640
Leu Glu His Phe Met Ser Lys Lys Ile Thr Leu Ile Pro Lys Ser Glu
                645                 650                 655
Ala Ala Asp Ser Asp Ile Val Asp Glu Asn Ala Gly Asp Phe Ile Val
            660                 665                 670
Lys Gln Glu Ser Met Leu Glu Ser
        675                 680


<210>  4
<211>  2043
<212>  DNA
<213>  Arabidopsis thaliana
<220>
<221>  CDS
<222>  (1)..(2040)
<223>
<400>  4
atg gca cga aat cgg gag ggt ttg gtt ttg gtg ctc gat gta ggt cct        48

Met Ala Arg Asn Arg Glu Gly Leu Val Leu Val Leu Asp Val Gly Pro
1               5                   10                  15
gcg atg cgt agt gtt ctc cct gat gtt gaa aag gca tgt tca atg cta      96
Ala Met Arg Ser Val Leu Pro Asp Val Glu Lys Ala Cys Ser Met Leu
            20                  25                  30
ctt cag aag aag ttg att tac aac aag tat gat gaa gtt gga att gtt      144
Leu Gln Lys Lys Leu Ile Tyr Asn Lys Tyr Asp Glu Val Gly Ile Val
        35                  40                  45
gtc ttt gga act gaa gaa act gga aac gag ctt gct agg gag ata ggt      192
Val Phe Gly Thr Glu Glu Thr Gly Asn Glu Leu Ala Arg Glu Ile Gly
    50                  55                  60
gga tat gag aat gtt aca gtt tta agg aat atc aga gtt gtt gat gaa      240
Gly Tyr Glu Asn Val Thr Val Leu Arg Asn Ile Arg Val Val Asp Glu
65                  70                  75                  80
tta gcg gca gag cat gtg aag cag ctt cct cga gga act gta gct gga      288
Leu Ala Ala Glu His Val Lys Gln Leu Pro Arg Gly Thr Val Ala Gly
                85                  90                  95
gac ttt ctt gat gct tta att gtt ggg atg gat atg ctt ata aag atg      336
Asp Phe Leu Asp Ala Leu Ile Val Gly Met Asp Met Leu Ile Lys Met
            100                 105                 110
tat ggt aat gca cac aag ggg aaa aaa cgg atg tgt ctc atc acg aac      384
Tyr Gly Asn Ala His Lys Gly Lys Lys Arg Met Cys Leu Ile Thr Asn
        115                 120                 125
gca gct tgt cct acc aaa gat cca ttt gaa ggg acg aaa gat gat caa      432
Ala Ala Cys Pro Thr Lys Asp Pro Phe Glu Gly Thr Lys Asp Asp Gln
    130                 135                 140
gtc agc act att gct atg aaa atg gct gcg gag gga att aag atg gag      480
Val Ser Thr Ile Ala Met Lys Met Ala Ala Glu Gly Ile Lys Met Glu
145                 150                 155                 160
agc att gtt atg agg tct aat tta agt ggt gat gct cat gag aga gta      528
Ser Ile Val Met Arg Ser Asn Leu Ser Gly Asp Ala His Glu Arg Val
                165                 170                 175
ata gag gag aat gat cat ctg ttg act tta ttc tcc agc aac gcg att      576
Ile Glu Glu Asn Asp His Leu Leu Thr Leu Phe Ser Ser Asn Ala Ile
            180                 185                 190
gcc aaa aca gtg aat gtt gat agt cca ctc tca ctg ctt ggt tcc tta      624
Ala Lys Thr Val Asn Val Asp Ser Pro Leu Ser Leu Leu Gly Ser Leu
        195                 200                 205
aag acc cgg agg gtg gct cca gtt act ttg ttc agg ggt gac ctt gaa      672
Lys Thr Arg Arg Val Ala Pro Val Thr Leu Phe Arg Gly Asp Leu Glu
    210                 215                 220
att aac cca aca atg aag att aag gtg tgg gtt tat aag aaa gtg gct      720
Ile Asn Pro Thr Met Lys Ile Lys Val Trp Val Tyr Lys Lys Val Ala
225                 230                 235                 240
gag gag aga ctt ccc act cta aaa atg tat tct gac aaa gcg cct cca      768
Glu Glu Arg Leu Pro Thr Leu Lys Met Tyr Ser Asp Lys Ala Pro Pro
                245                 250                 255
act gac aag ttt gcc aaa cat gaa gta aaa gtt gat tat gat tac aaa      816
Thr Asp Lys Phe Ala Lys His Glu Val Lys Val Asp Tyr Asp Tyr Lys
            260                 265                 270
gtt act gca gaa tct act gag gtt att gct cca gaa gaa agg atc aag      864
Val Thr Ala Glu Ser Thr Glu Val Ile Ala Pro Glu Glu Arg Ile Lys
        275                 280                 285
ggc ttt cgc tat gga cct cag gtt att cct ata tca cca gat cag ata      912
Gly Phe Arg Tyr Gly Pro Gln Val Ile Pro Ile Ser Pro Asp Gln Ile
    290                 295                 300
gaa aca ctc aag ttt aaa act gac aaa ggc atg aag ctt ctg gga ttt      960
Glu Thr Leu Lys Phe Lys Thr Asp Lys Gly Met Lys Leu Leu Gly Phe
305                 310                 315                 320
act gag gcg tca aat ata ctg cga cat tat tac atg aaa gac gtg aat      1008
Thr Glu Ala Ser Asn Ile Leu Arg His Tyr Tyr Met Lys Asp Val Asn
                325                 330                 335
ata gtt gtt cct gat cca agc aaa gaa aaa tct gta ctt gcc gta tct      1056
Ile Val Val Pro Asp Pro Ser Lys Glu Lys Ser Val Leu Ala Val Ser
            340                 345                 350

gct att gca aga gaa atg aaa gaa aca aac aag gta gca att gta cga 1104
Ala Ile Ala Arg Glu Met Lys Glu Thr Asn Lys Val Ala Ile Val Arg
        355                 360                 365

tgt gtc tgg aga aat gga caa ggc aat gtg gtt gtt ggg gtc ctg act 1152
Cys Val Trp Arg Asn Gly Gln Gly Asn Val Val Val Gly Val Leu Thr
    370                 375                 380

cca aat gtg tct gaa aga gat gat act cct gat tcg ttt tac ttc aat 1200
Pro Asn Val Ser Glu Arg Asp Asp Thr Pro Asp Ser Phe Tyr Phe Asn
385                 390                 395                 400

gtg cta cct ttc gcc gag gat gtc cgc gag ttt cca ttt cct tct ttc 1248
Val Leu Pro Phe Ala Glu Asp Val Arg Glu Phe Pro Phe Pro Ser Phe
                405                 410                 415

aac aag ctt cct tca tca tgg aag cca gat gaa caa caa caa gca gta 1296
Asn Lys Leu Pro Ser Ser Trp Lys Pro Asp Glu Gln Gln Gln Ala Val
            420                 425                 430

gca gat aat ttg gtt aag atg ctt gac ctt gca cct tct gcg gaa gag 1344
Ala Asp Asn Leu Val Lys Met Leu Asp Leu Ala Pro Ser Ala Glu Glu
        435                 440                 445

gaa gta cta aag cct gat ctt act cct aac ccg gtt ttg cag cgc ttt 1392
Glu Val Leu Lys Pro Asp Leu Thr Pro Asn Pro Val Leu Gln Arg Phe
    450                 455                 460

tat gaa tac ctt gag ttg aaa tcg aaa tcc acg gat gct act tta cct 1440
Tyr Glu Tyr Leu Glu Leu Lys Ser Lys Ser Thr Asp Ala Thr Leu Pro
465                 470                 475                 480

cca atg gat gga act ttc aag aga ctt atg gag cag gac cct gaa ctc 1488
Pro Met Asp Gly Thr Phe Lys Arg Leu Met Glu Gln Asp Pro Glu Leu
                485                 490                 495

tct tcc aac aat aaa tcc ata atg gac acg ttt cgt gga agt ttt gaa 1536
Ser Ser Asn Asn Lys Ser Ile Met Asp Thr Phe Arg Gly Ser Phe Glu
            500                 505                 510

gtc aag gag aat cca aag ttg aag aag gct tct aag aga ttg ttg cgg 1584
Val Lys Glu Asn Pro Lys Leu Lys Lys Ala Ser Lys Arg Leu Leu Arg
        515                 520                 525

gat aaa cca tcg ggt tca gat gat gaa gac aat cgc atg att act tat 1632
Asp Lys Pro Ser Gly Ser Asp Asp Glu Asp Asn Arg Met Ile Thr Tyr
    530                 535                 540

gac gcc aaa gag aac aaa att gat ata gtc gga gat gca aac cca att 1680
Asp Ala Lys Glu Asn Lys Ile Asp Ile Val Gly Asp Ala Asn Pro Ile
545                 550                 555                 560

caa gat ttt gaa gcc atg ata tcc cgc aga gat aaa act gac tgg acc 1728
Gln Asp Phe Glu Ala Met Ile Ser Arg Arg Asp Lys Thr Asp Trp Thr
                565                 570                 575

gag aaa gca atc aca caa atg aaa aac cta ata atg aag ctt gta gaa 1776
Glu Lys Ala Ile Thr Gln Met Lys Asn Leu Ile Met Lys Leu Val Glu
            580                 585                 590

aat tgt acc gac gag ggt gac aaa gca ttg gaa tgt gta ctc gct ctt 1824
Asn Cys Thr Asp Glu Gly Asp Lys Ala Leu Glu Cys Val Leu Ala Leu
        595                 600                 605

cgt aaa ggc tgc gtc ttg gag cag gag cca aag caa ttc aat gag ttc 1872
Arg Lys Gly Cys Val Leu Glu Gln Glu Pro Lys Gln Phe Asn Glu Phe
    610                 615                 620

ttg aac cat cta ttc aag tta tgc caa gaa aga aac tta tct cat ctt 1920
Leu Asn His Leu Phe Lys Leu Cys Gln Glu Arg Asn Leu Ser His Leu
625                 630                 635                 640

ctc gaa cat ttc atg tca aag aag atc act ttg att ccc aaa tcc gaa 1968
Leu Glu His Phe Met Ser Lys Lys Ile Thr Leu Ile Pro Lys Ser Glu
                645                 650                 655

gca gcg gac agt gat ata gta gac gag aac gct ggg gat ttc atc gtt 2016
Ala Ala Asp Ser Asp Ile Val Asp Glu Asn Ala Gly Asp Phe Ile Val
            660                 665                 670

aaa caa gag tca atg ctc gag agc taa 2043
Lys Gln Glu Ser Met Leu Glu Ser
        675                 680

<210>  5
<211>  23
<212>  DNA
<213>  consensus sequence
<220>
<221>  misc_feature
<222>  (1)..(23)
<223>  consensus target sites of ZFNs
<220>
<221>  misc_feature
<222>  (1)..(2)
<223>  n = a, c, g, or t
<220>
<221>  misc_feature
<222>  (4)..(5)
<223>  n = a, c, g, or t
<220>
<221>  misc_feature
<222>  (7)..(8)
<223>  n = a, c, g, or t
<220>
<221>  misc_feature
<222>  (10)..(14)
<223>  n = a, c, g, or t
<220>
<221>  misc_feature
<222>  (16)..(17)
<223>  n = a, c, g, or t
<220>
<221>  misc_feature
<222>  (19)..(20)
<223>  n = a, c, g, or t
<220>
<221>  misc_feature
<222>  (22)..(23)
<223>  n = a, c, g, or t
<400>  5
nncnncnncn nnnngnngnn gnn                                              23


<210>  6
<211>  7
<212>  PRT
<213>  Artificial
<220>
<223>  An artificial zinc finger sequence
<400>  6
Gln Arg Ala His Leu Glu Arg
1               5


<210>  7
<211>  7
<212>  PRT
<213>  Artificial
<220>
<223>  An artificial zinc finger sequence
<400>  7
Gln Ser Gly His Leu Gln Arg
1               5


<210>  8
<211>  7
<212>  PRT

<213>  Artificial
<220>
<223>  An artificial zinc finger sequence
<400>  8
Gln Arg Ala His Leu Glu Arg
1               5


<210>  9
<211>  7
<212>  PRT
<213>  Artificial
<220>
<223>  An artificial zinc finger sequence
<400>  9
Arg Ser Asp Ala Leu Thr Arg
1               5


<210>  10
<211>  7
<212>  PRT
<213>  Artificial
<220>
<223>  An artificial zinc finger sequence
<400>  10
Arg Ser Asp Asp Leu Gln Arg
1               5


<210>  11
<211>  7
<212>  PRT
<213>  Artificial
<220>
<223>  An artificial zinc finger sequence
<400>  11
Arg Ser Asp Asp Leu Gln Arg
1               5


<210>  12
<211>  105
<212>  DNA
<213>  Artificial
<220>
<223>  An artificial sequence encoding a zinc finger
<400>  12
ctagacctgg agaaaagcct tatgcttgcc ctgtggagtc ctgcgatagg agattttctc    60
agtccggcaa cctcgtgagg catattcgta tccataccgg tggtg                    105


<210>  13
<211>  105
<212>  DNA
<213>  Artificial
<220>
<223>  An artificial sequence encoding a zinc finger
<400>  13
gatccaccac cggtatggat acgaatatgc ctcacgaggt tgccggactg agaaaatctc    60
ctatcgcagg actccacagg gcaagcataa ggcttttctc caggt                    105


<210>  14
<211>  105

<212>  DNA
<213>  Artificial
<220>
<223>  An artificial sequence encoding a zinc finger
<400>  14
ctagacctgg agaaaagcct tatgcttgcc ctgtggagtc ctgcgatagg agattttcta     60
ggtccgacgc tctcactagg catatccgta tccataccgg tggtg                    105


<210>  15
<211>  105
<212>  DNA
<213>  Artificial
<220>
<223>  An artificial sequence encoding a zinc finger
<400>  15
gatccaccac cggtatggat acggatatgc ctagtgagag cgtcggacct agaaaatctc     60
ctatcgcagg actccacagg gcaagcataa ggcttttctc caggt                    105


<210>  16
<211>  93
<212>  DNA
<213>  Artificial
<220>
<223>  An artificial sequence encoding a zinc finger
<400>  16
ccgggcagaa gcctttccag tgtcgtatct gcatgaggaa cttcagtagg tccgacaacc     60
tcgccaggca catccgtact cacaccggtg gtg                                  93


<210>  17
<211>  93
<212>  DNA
<213>  Artificial
<220>
<223>  An artificial sequence encoding a zinc finger
<400>  17
gatccaccac cggtgtgagt acggatgtgc ctggcgaggt tgtcggacct actgaagttc     60
ctcatgcaga tacgacactg gaaaggcttc tgc                                  93


<210>  18
<211>  93
<212>  DNA
<213>  Artificial
<220>
<223>  An artificial sequence encoding a zinc finger
<400>  18
ccgggcagaa gcctttccag tgtcgtatct gcatgaggaa cttcagtagg tccgacgacc     60
tccagaggca catccgtact cacaccggtg gtg                                  93


<210>  19
<211>  93
<212>  DNA
<213>  Artificial
<220>
<223>  An artificial sequence encoding a zinc finger
<400>  19
gatccaccac cggtgtgagt acggatgtgc ctctggaggt cgtcggacct actgaagttc     60
ctcatgcaga tacgacactg gaaaggcttc tgc                                  93


<210>  20

<211>  90
<212>  DNA
<213>  Artificial
<220>
<223>  An artificial sequence encoding a zinc finger
<400>  20
ccggggagaa gcctttcgcc tgtgacattt gtgggaggaa gttcgctagg tccgacaacc      60
tcgccaggca taccaaaatc catactggtg                                        90

<210>  21
<211>  90
<212>  DNA
<213>  Artificial
<220>
<223>  An artificial sequence encoding a zinc finger
<400>  21
gatccaccag tatggatttt ggtatgcctg gcgaggttgt cggacctagc gaacttcctc      60
ccacaaatgt cacaggcgaa aggcttctcc                                        90

<210>  22
<211>  90
<212>  DNA
<213>  Artificial
<220>
<223>  An artificial sequence encoding a zinc finger
<400>  22
ccggggagaa gcctttcgcc tgtgacattt gtgggaggaa gttcgctagg tccgacgacc      60
tcactaggca taccaaaatc catactggtg                                        90

<210>  23
<211>  90
<212>  DNA
<213>  Artificial
<220>
<223>  An artificial sequence encoding a zinc finger
<400>  23
gatccaccag tatggatttt ggtatgccta gtgaggtcgt cggacctagc gaacttcctc      60
ccacaaatgt cacaggcgaa aggcttctcc                                        90

<210>  24
<211>  30
<212>  DNA
<213>  Artificial
<220>
<223>  An artificially synthesized primer sequence
<400>  24
tggacccttt agcttcccaa catcaacaca                                        30

<210>  25
<211>  30
<212>  DNA
<213>  Artificial
<220>
<223>  An artificially synthesized primer sequence
<400>  25
accggaacat cagtgagctc gatgtcagaa                                        30

## Description

### [Technical Field]

The present invention relates to a method for producing a plant cell in which a mutation is introduced at a specific target DNA site on a chromosome. More specifically, the present invention relates to a method for producing a plant cell in which a mutation is introduced at a target DNA site on a chromosome through DNA double-strand break with a restriction endonuclease and nonhomologous end joining, wherein a plant cell in which a function of a protein involved in repair of a broken double-stranded DNA is suppressed is used.

### [Background Art]

A major focus of plant biotechnology is genetic modification and improvement of crop plants. To this object, large-scale genome analyses have been conducted for a model plant Arabidopsis, and also for many crop plants such as rice, maize, wheat, soybean, and tomato. As a result, a vast amount of genome-sequence information has been available, which intensifies the need for development of a method capable of directly modifying target genes on genomes of higher plants on the basis of the sequence information. However, no efficient and versatile methods have been developed so far.

The most widely used strategy for site-specific mutagenesis is gene targeting utilizing homologous recombination. Efficient gene targeting methods have been used for yeast (NPL 1) and mice (NPL 2) for 20 years or more. Further, gene targeting has been conducted in Arabidopsis and rice (NPLs 3 to 6). Typically, gene targeting events occur in a small proportion of treated cells (10⁻⁶ to 10⁻² gene-targeting events per cell of yeast, and 10⁻⁷ to 10⁻⁵ gene-targeting events per ES cell of mice). The gene targeting efficiency per cell in higher plants is extremely low (less than 10⁻⁷ gene targeting events per cell) (NPLs 3 and 7 to 11).

Such a low gene targeting frequency in higher plants is thought to result from competition between homologous recombination and nonhomologous end joining (NHEJ) for DNA double-strand break repair. The main pathway of double-strand break repair seems to be nonhomologous end joining (NPLs 12 and 13). Consequently, nonhomologous end joining seems more likely to be involved in the ends of donor molecules than homologous recombination does, so that the gene targeting efficiency is reduced. Data covering a wide range have suggested that double-strand break repair by nonhomologous end joining in higher plants is error-prone. Double-strand breaks are often repaired by nonhomologous end joining processes that involve insertions and/or deletions (NPLs 14 and 15). When these observation results are taken together, it is suggested that nonhomologous end joining-based strategies for targeted mutagenesis in higher plants are more efficient than homologous recombination-based strategies. Indeed, expression of I-SceI, which is a restriction enzyme causing cleavage on a chromosome at a low frequency, has been shown to induce mutations at I-SceI cleavage sites in Arabidopsis and tobacco (NPL 16). Nevertheless, the use of the enzyme is limited to rarely occurring natural recognition sites or artificial target sites.

To overcome this problem, zinc finger nucleases (ZFNs) have been developed. Zinc finger nucleases are chimeric proteins comprising a zinc finger-based artificial binding domain and a DNA cleavage domain. By modification of DNA binding sites, a zinc finger nuclease can be specially designed to cleave a double-stranded DNA sequence with virtually any length (NPLs 17 and 18).

Nonhomologous end joining-based targeted mutagenesis strategies have been developed recently for several organisms by using artificial zinc finger nucleases to generate double-strand breaks at specific genomic sites (NPLs 19 to 23). Repair of double-strand breaks by nonhomologous end joining frequently produces deletions and/or insertions at binding sites. Two groups have successfully used zinc finger nucleases to genetically mutate genes in embryos of zebrafish. Specific zinc finger motifs have been designed to recognize distinct DNA sequences (NPLs 19 and 20). An mRNA encoding a zinc finger nuclease was introduced into one-cell embryos and a high percentage of animals carried desired mutations and phenotypes. These studies demonstrated that the use of zinc finger nucleases makes it possible to specifically and efficiently create genetic mutations at desired loci in a germ line, and the zinc finger nuclease-induced alleles are transferred to subsequent generations.

Lloyd et al., (NPL 21) used zinc finger nucleases for site-specific mutagenesis in Arabidopsis, and reported that, as expected, induction of zinc finger nuclease expression caused mutations at target sites on chromosomes in seedlings of the subsequent generations at a frequency of 7.9%. However, this report uses a model system using an artificial target site for a previously reported 3-finger-type ZFN_QQR (NPL 24). For this reason, it remains uncertain whether or not zinc finger nucleases generate breaks efficiently, and induce mutations at target sites in endogenous genes of Arabidopsis. In addition, it was very recently reported that zinc finger nucleases can induce mutations at target sites in endogenous genes in maize and tobacco (NPLs 25 and 26), but the possibility of improvement in mutation introduction efficiency is not reported.

### [Citation List]

### [Patent Literature]

[PTL 1] Japanese Unexamined Patent Application Publication No. 2005-237316

### [Non Patent Literature]

[NPL 1] Rothstein R, Methods Enzymol 194: 281-301, 1991
[NPL 2] Capecci MR, Science 244: 1288-1292, 1989
[NPL 3] Hanin M et al., Plant J 28: 671-677, 2001
[NPL 4] Halfter U et al., Mol Gen Genet 231: 186-193, 1992
[NPL 5] Hrouda M & Paszkowski, J Mol Gen Genet 243: 106-111, 1994
[NPL 6] Lee KY et al., Plant Cell 2: 415-425, 1990
[NPL 7] Offringa R et al., EMBO J 9: 3077-3084, 1990
[NPL 8] Paszkowski J et al., EMBO J 7: 4021-4026, 1988
[NPL 9] Terada R et al., Nat Biotechnol 20: 1030-1034, 2002
[NPL 10] Endo M, Osakabe K, Ichikawa H, Toki S, Plant Cell Physiol 47: 372-379, 2006
[NPL 11] Endo M et al., Plant J 52: 157-166, 2007
[NPL 12] Ray A & Langer M, Trends Plant Sci 7: 435-440, 2002
[NPL 13] Britt AB & May GD, Trends Plant Sci 8: 90-95, 2003
[NPL 14] Gorbunova VV & Levy AA, Trends Plant Sci 4 : 263-269, 1999
[NPL 15] Britt AB, Trend in Plant Sci 4: 20-25, 1999
[NPL 16] Kirik A et al., EMBO J 19: 5562-5566, 2000
[NPL 17] Kim YG et al., Proc Natl Acad Sci USA 93: 1156-1160, 1996
[NPL 18] Cathomen T & Joung JK, Mol Ther 16: 1200-1207, 2008
[NPL 19] Doyon Y et al., Nat Biotechnol 26: 702-708, 2008
[NPL 20] Meng X et al., Nat Biotechnol 26: 695-701, 2008
[NPL 21] Lloyd A et al., Proc Natl Acad Sci USA 102: 2232-2237, 2005
[NPL 22] Beumer KJ et al., Proc Natl Acad Sci USA 105: 19821-19826, 2008
[NPL 23] Santiago Y et al., Proc Natl Acad Sci USA 105: 5809-5814, 2008
[NPL 24] Smith J et al., Nucleic Acids Res 28: 3361-3369, 2000
[NPL 25] Shukla VK et al., Nature 459: 437-441, 2009
[NPL 26] Townsend JA et al., Nature 459: 442-445, 2009

### [Summary of Invention]

### [Technical Problem]

The present invention has been made in view of such circumstances, and an object of the present invention is to provide a method for producing a plant cell in which a mutation is introduced at a specific target DNA site on a chromosome by use of a restriction endonuclease, the method being capable of effectively introducing the mutation.

### [Solution to Problem]

First, in order to investigate whether or not DNA double-strand break with a zinc finger nuclease and subsequent repair by nonhomologous end joining occur even when a zinc finger nuclease target DNA site is present on an endogenous gene of a plant, the present inventors specially designed a zinc finger nuclease having a target DNA site on an endogenous gene of Arabidopsis. Then, a vector which expresses this zinc finger nuclease was introduced into Arabidopsis, double-strand break was induced at the target DNA site, and mutations at the target DNA site were detected. As a result, the present inventors has found that mutations are introduced even on the endogenous gene of Arabidopsis through double-strand break at the target DNA site on the endogenous gene and subsequent repair by nonhomologous end joining. However, it was revealed that most of mutations at the target DNA site are silent mutations or point mutations, which do not bring about change in the amino acid sequences of the encoded protein, and do not exert influence on the phenotype. For the purpose of analyzing a function of a plant gene by introducing a mutation, or the purpose of producing a crop plant having a useful trait by genetically improving a crop plant, it is not preferable that silent mutations occur at a high frequency in mutations introduced. Moreover, in the case of point mutations, reverse mutations may occur. Hence, it is not preferable that the mutations introduced are point mutations from the viewpoint of stability of mutations.

In this respect, the present inventors next have made earnest study on a method for increasing the frequency of mutations which are neither non-silent mutations nor point mutations. Here, the present inventors have hypothesized that activities of proteins involved in repair by nonhomologous end joining inhibit introductions of drastic mutations such as large deletions and/or insertions at DNA double-strand break ends. On this hypothesis, the present inventors induced double-strand breaks at target DNA sites with a zinc finger nuclease by use of cells in which these functions of the proteins are suppressed, and investigated mutations introduced by subsequent nonhomologous end joining. As a result, the suppression of the functions of the proteins did not leads to change in the frequency of mutations caused by nonhomologous end joining after DNA double-strand break, and it was revealed that most of the mutations produced were mutations involving deletions which are highly likely to exert an influence on a phenotype of Arabidopsis.

So far, it has been reported that, by suppressing functions of proteins necessary for nonhomologous end joining, the frequency of nonhomologous recombination is reduced, so that the frequency of homologous recombination is improved (PTL 1). In contrast, the present inventors elucidated that, even when these functions of the proteins are suppressed, not only the frequency of non-fidelity nonhomologous end joining, which is nonhomologous recombination, is not reduced, but also the quality of mutations introduced by nonhomologous end joining is drastically changed.

Specifically, the present inventors have found that DNA double-strand break with a restriction endonuclease can be performed even when a target DNA site is present on an endogenous gene in a plant, and also found that, by use of a plant cell in which a function of a protein involved in repair by nonhomologous end joining is artificially suppressed, the frequency of non-silent mutations can be dramatically increased in a repairing process of a broken double-stranded DNA by nonhomologous end joining. These findings have lead to the completion of the present invention.

Accordingly, the present invention relates to a method for producing a plant cell in which a mutation is introduced at a specific target DNA site on a chromosome by use of a restriction endonuclease such as a zinc finger nuclease, the method using a plant cell in which a function of a protein involved in nonhomologous end joining is artificially suppressed, and also relates to a plant cell produced by the method and a plant comprising the plant cell. More specifically, the following invention is provided.
(1) A method for producing a genetically modified plant cell, comprising:
   expressing a restriction endonuclease in a plant cell in which a function of a protein involved in repair by nonhomologous end joining is artificially suppressed, thereby inducing a double-strand break at a target DNA site of the restriction endonuclease on a chromosome of the plant cell; and
   causing a mutation at the target DNA site through a repairing process by nonhomologous end joining at the broken target DNA site of the plant cell.
(2) The method according to (1), wherein the protein involved in the repair by the nonhomologous end joining is at least one of Ku70 and Ku80.
(3) The method according to (1) or (2), wherein the restriction endonuclease is a zinc finger nuclease.
(4) A genetically modified plant cell which is produced by the method according to any one of (1) to (3).
(5) A plant comprising the cell according to (4).
(6) A plant which is a progeny or a clone of the plant according to (5).
(7) A propagation material of the plant according to (5) or (6).
(8) A kit for use in the method according to any one of (1) to (3), comprising at least one of the following (a) to (c):
   (a) a plant cell in which a function of a protein involved in repair of a broken double-stranded DNA is artificially suppressed;
   (b) a DNA construct for artificially suppressing a function of a protein involved in repair of a broken double-stranded DNA in a plant cell; and
   (c) a DNA construct for expressing a restriction endonuclease in a plant cell.

### [Advantageous Effects of Invention]

So far, it has been known that mutations can be introduced into target genes of higher plants by a restriction endonuclease treatment. However, most of mutations introduced are one-base substitutions, which are often silent mutations where the mutations are not expressed in the phenotype. Moreover, when the mutations are one-base substitutions, reverse mutations may occur. In contrast, the method of the present invention using a plant cell in which a function of a protein involved in nonhomologous end joining is artificially suppressed makes it possible to generate several-base to about-10-base deletions at a high frequency at a cleavage site of a restriction endonuclease. Accordingly, the method of the present invention makes it possible to efficiently perform direct induction of mutations which are highly likely to be expressed in the phenotype of a plant.

### [Brief Description of Drawings]

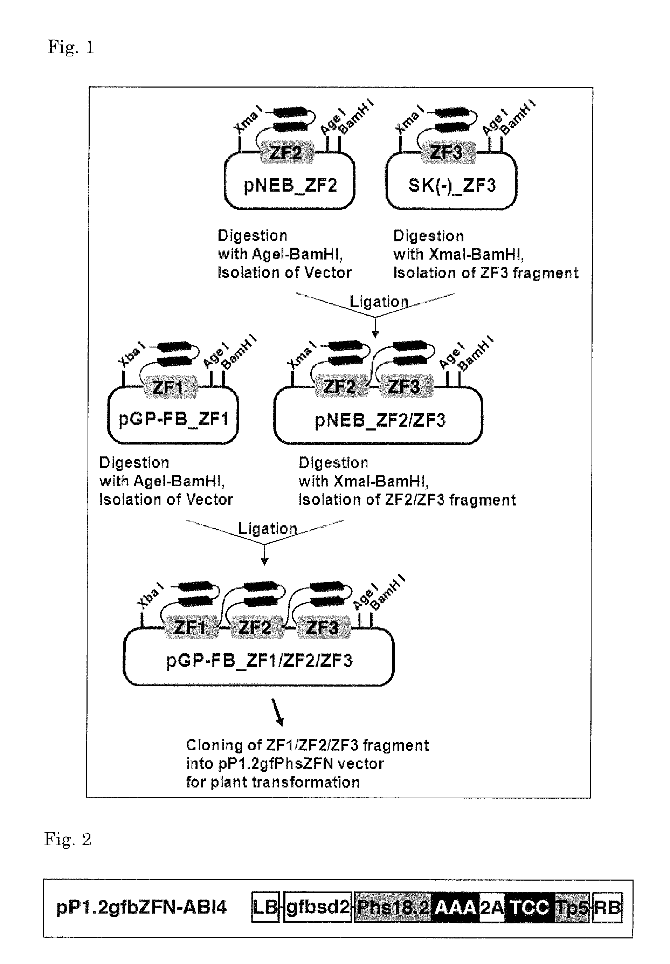

[Fig. 1] Fig. 1 is a diagram showing a construction process of a zinc finger nuclease expression vector.
[Fig. 2] Fig. 2 is a diagram showing T-DNA of a zinc finger nuclease expression binary vector. In this figure, "LB" represents a left border sequence, "RB" represents a right border sequence, "gfbsd2" represents a fused gene of GFP and a blasticidin S resistance gene, a gfbsd2 expression cassette driven by a 2x cauliflower mosaic virus 35S gene promoter, and a nopaline synthase gene terminator, "Phsp18.2" represents an Arabidopsis HSP18.2 gene promoter, and "Tp5" represents an Arabidopsis polyA-binding protein PAB5 gene terminator.

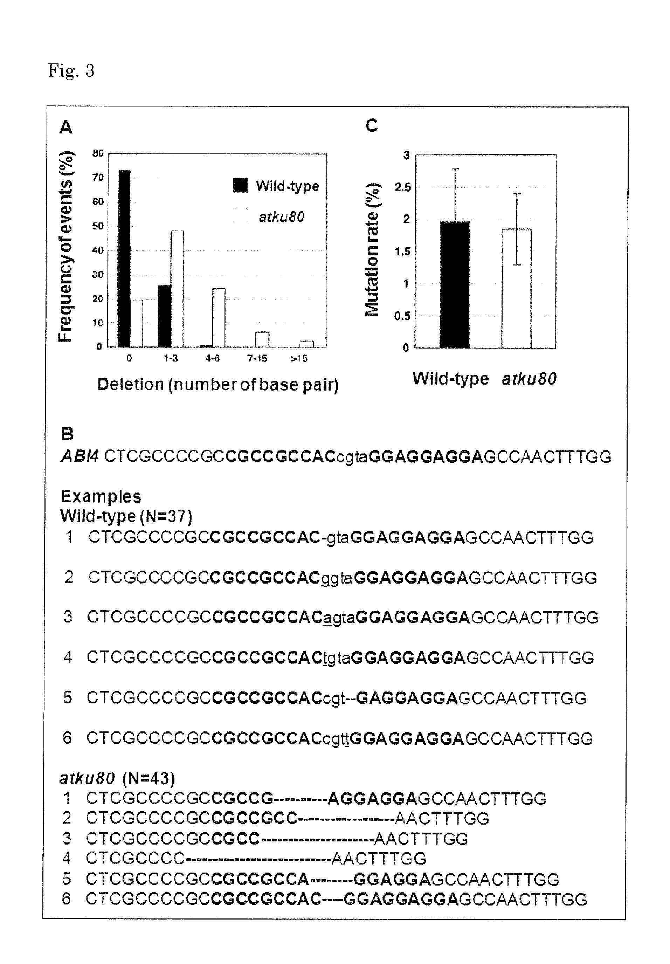

[Fig. 3] Fig. 3 shows diagrams showing the frequency and fidelity of DNA repaired by nonhomologous end joining after break with zinc finger nucleases in the ABI4 gene in atku80 cells. (A) shows a distribution of length of deletions at junctions, where a deletion is the sum of base pairs lost at sites on the both sides of a DNA double-strand break. (B) shows examples of repaired DNA sequences obtained from genomic DNA of wild-type and atku80 plants after break with zinc finger nuclease, where zinc finger nuclease recognition sites are shown in bold, putative break sites are shown in lower case, mutations are represented by underlines, and deletions are represented by hyphens. (C) shows the mutation rate after induction of DNA double-strand breaks with zinc finger nucleases. The relative frequency of non-fidelity end joining was calculated from the number of DNA clones positive for SURVEYOR nuclease assay per number of DNA clones tested. The experiment was repeated three times, and data are expressed as mean ± SD.

### [Description of Embodiments]

The present invention is based on findings by the present inventors that, in producing a plant cell in which a mutation is introduced at a specific target DNA site on a chromosome through DNA double-strand break induced by a restriction endonuclease, the use of a plant cell in which a function of a protein involved in repair by nonhomologous end joining is suppressed results in drastic change in quality of a mutation introduced.

The method for producing a genetically modified plant cell of the present invention comprises: expressing a restriction endonuclease in a plant cell in which a function of a protein involved in repair by nonhomologous end joining is artificially suppressed, thereby inducing a double-strand break at a target DNA site of the restriction endonuclease on a chromosome of the plant cell; and causing a mutation at the target DNA site through a repairing process by nonhomologous end joining at the broken target DNA site of the plant cell.

The use of the plant cell in which a function of a protein involved in repair by nonhomologous end joining is suppressed makes it possible to dramatically increase the frequency of non-silent mutation in the repairing process by nonhomologous end joining at the target DNA site subjected to a double-strand break. The present invention makes it possible to increase the frequency of non-silent mutation generally twice or more, and preferably 2.5 times or more (for example, approximately 2.7 times), as compared with a wild-type control (a case where a function of a protein involved in repair by nonhomologous end joining is not suppressed). Non-silent mutations introduced at target DNA sites by the method of the present invention are typically base deletions. As shown in Fig. 3, the method of the present invention makes it possible to improve the frequencies of introductions of 1- to 3-base deletions, 4- to 6-base deletions, 7- to 15-base deletions, and 15 or more-base deletions at the target DNA site, as compared with the case of the wild type. The present invention makes it possible to dramatically improve the frequency of introductions of deletions of particularly 4 bases or more, as compared with the case of the wild type.

In the present invention, the "protein involved in repair by nonhomologous end joining" which is the target of suppression of the function is not particularly limited, as long as the suppression of the function of the protein enables the frequency of the above-described non-silent mutations to be increased in the repairing process by nonhomologous end joining at the target DNA site subjected to the double-strand break. The protein is preferably Ku70 or Ku80. Note that Ku70 and Ku80 genes from Arabidopsis are disclosed in a document (Tamura K, et al. Plant J, 29: 771-781, 2002), and Ku70 and Ku80 genes from Mung bean are disclosed in a document (Liu PF, et al. Biochem Biophys Acta, 1769: 443-454, 2007). The amino acid sequence of Ku70 from Arabidopsis is shown in SEQ ID NO: 1, the base sequence of DNA encoding the amino acid sequence is shown in SEQ ID NO: 2. Meanwhile, the amino acid sequence of Ku80 from Arabidopsis is shown in SEQ ID NO: 3, and the base sequence of DNA encoding the amino acid sequence is shown in SEQ ID NO: 4.

In the present invention, the meaning of "suppression of a function of a protein" includes both complete suppression and partial suppression of a function of a protein. As a method for suppressing the function of the protein, it is possible to use a method well-known to those skilled in the art, such as a method using the RNA interference technology, a method using the antisense technology, a method using the ribozyme technology, or a method of disrupting a target gene.

In the method using RNA interference, DNA encoding dsRNA (double-stranded RNA) complementary to a transcription product of a target gene is used. When dsRNA of approximately 40 to several hundred base pairs is introduced into a cell, the dsRNA is excised from the 3' end into pieces each comprising approximately 21 to 23 base pairs with an RNase III-like nuclease, called a dicer, having a helicase domain in the presence of ATP, so that siRNA (short interference RNA) is formed. Specific proteins bind to the siRNA to form a nuclease complex (RISC: RNA-induced silencing complex). The complex recognizes and binds to a sequence which is the same as that of the siRNA, and cleaves the transcription product (mRNA) of the target gene at a central portion of the siRNA by an RNase III-like enzymatic activity. In addition to this pathway, the antisense strand of the siRNA binds to mRNA, and acts as a primer for a RNA-dependant RNA polymerase (RsRP) to synthesize dsRNA. A pathway is also considered in which this dsRNA serves as a substrate of the dicer again to form new siRNA, so that the action is amplified.

The DNA encoding dsRNA comprises an antisense DNA which encodes an antisense RNA corresponding to any region of the transcription product (mRNA) of the target gene, and a sense DNA which encodes a sense RNA of any region of the mRNA, and the antisense RNA and the sense RNA can be expressed from the antisense DNA and the sense DNA, respectively. In addition, the dsRNA can be prepared from these antisense RNA and sense RNA.

When an sRNA expression system is incorporated into a vector or the like, it is possible to employ a structure with which the antisense RNA and the sense RNA are expressed from the same vector, or a structure with which the antisense RNA and the sense RNA are expressed from different vectors, respectively. In an example of the structure in which the antisense RNA and the sense RNA are expressed from the same vector, an antisense RNA expression cassette and a sense RNA expression cassette are individually assembled in which promoters capable of expressing a short RNA, such as the polIII system, are linked upstream of the antisense DNA and the sense DNA, respectively, and these cassettes are inserted into a vector in the same direction or in opposite directions.

Moreover, the expression system may also have a structure in which the antisense DNA and the sense DNA are arranged in opposite directions so as to face each other on different strands. This structure comprises one double-stranded DNA (siRNA-encoding DNA) in which an antisense RNA-encoding strand and a sense RNA-encoding strand are paired, and promoters on both sides of the double-stranded DNA in opposite directions, so that the antisense RNA and the sense RNA can be expressed from the strands, respectively. In this case, to avoid addition of an unnecessary sequence downstream of the sense RNA or the antisense RNA, a terminator is preferably provided to the 3' end of each of the strands (the antisense RNA-encoding strand and the sense RNA-encoding strand). A sequence of four or more consecutive A (adenine) bases or the like can be used as the terminator. Furthermore, the types of the two promoters are preferably different in this palindrome style expression system.

In an example of the structure with which the antisense RNA and the sense RNA are expressed from different vectors, an antisense RNA expression cassette and a sense RNA expression cassette are individually assembled in which promoters capable of expressing a short RNA, such as the polIII system, are linked upstream of the antisense DNA and the sense DNA, respectively, and these cassettes are incorporated into different vectors.

The dsRNA used in the present invention is preferably siRNA. The siRNA means a double-stranded RNA comprising strands short enough not to exhibit toxicity within a cell. The strand length of the siRNA is not particularly limited, as long as the siRNA can suppress the expression of the target gene and does not exhibit toxicity. The strand length of the dsRNA is, for example, 15 to 49 base pairs, preferably 15 to 35 base pairs, and further preferably 21 to 30 base pairs.

As the DNA encoding the dsRNA, it is also possible to use a construct which forms a double-stranded RNA having a hairpin structure (self-complementary 'hairpin' RNA (hpRNA)) upon insertion of an appropriate sequence (preferably an intron sequence) between inverted repeats of the target sequence (Smith, N.A., et al. Nature, 407: 319, 2000, Wesley, S. V. et al. Plant J. 27: 581, 2001, Piccin, A. et al. Nucleic Acids Res. 29: E55, 2001).

The DNA encoding the dsRNA does not have to be completely identical to the base sequence of the target gene, but has a sequence identity of at least 70% or more, preferably 80% or more, further preferably 90% or more (for example, 95%, 96%, 97%, 98%, 99% or more). The sequence identity can be determined by the BLAST program.

In the dsRNA, the double-stranded RNA portion in which RNAs are paired is not limited to those in which RNAs are completely paired, but may include unpaired portions due to mismatch (where corresponding bases are not complementary), bulge (where one of the strands lacks corresponding bases), or the like. In the present invention, the double-stranded RNA region in which the RNAs of the dsRNA are pared may include both bulge and mismatch.

Meanwhile, in the method using the antisense technology, a DNA (antisense DNA) encoding an antisense RNA complementary to the transcription product of the target gene is used. Actions of the antisense DNA to suppress the expression of the target gene include inhibition of transcription initiation by triple strand formation; suppression of transcription by hybrid formation at a site where RNA polymerase has formed a local open loop structure; inhibition of transcription by hybrid formation with RNA being synthesized; suppression of splicing by hybrid formation at a junction between an intron and an exon; suppression of splicing by hybrid formation at a spliceosome formation site; suppression of translocation from the nucleus to the cytoplasm by hybrid formation with mRNA; suppression of splicing by hybrid formation at a capping site or a poly (A) addition site; suppression of translation initiation by hybrid formation at a binding site of a translation initiation factor; suppression of translation by hybrid formation at a ribosome binding site near the start codon; inhibition of peptide chain elongation by hybrid formation in a translation region or at a polysome binding site of mRNA; suppression of gene expression by hybrid formation at a site of interaction between a nucleic acid and a protein; and the like. These suppress the expression of the target gene by inhibiting the process of transcription, splicing, or translation (Hirashima and Inoue, "Shin Seikagaku Jikken Koza (New Biochemistry Experimentation Lectures) 2, Kakusan (Nucleic Acid) IV, Idenshi No Fukusei To Hatsugen (Replication and Expression of Genes)," edited by Nihon Seikagakukai (The Japanese Biochemical Society), Tokyo Kagaku Doj in Co., ltd., pp. 319-347, 1993). The antisense DNA used in the present invention may suppress the expression of the target gene by any one of the above-described actions. In one embodiment, a design of an antisense sequence complementary to an untranslated region near the 5' end of mRNA of the target gene will be effective for inhibition of gene translation. Alternatively, a sequence complementary to a coding region or an untranslated region on the 3' side may also be used. Thus, the antisense DNA used in the present invention encompasses DNAs comprising antisense sequences of sequences in not only translation regions but also untranslated regions of a gene. The antisense DNA to be used is linked downstream of an appropriate promoter, and preferably a sequence comprising a transcription termination signal is linked on the 3' side of the antisense DNA.

The antisense DNA can be prepared based on the sequence information of the target gene by the phosphorothioate method (Stein, Nucleic Acids Res., 16: 3209-3221, 1988) or the like.
The sequence of the antisense DNA is preferably a sequence complementary to the transcription product of the target gene, but does not have to be completely complementary, as long as the gene expression can be inhibited effectively. The transcribed RNA is preferably 90% or more (for example, 95%, 96%, 97%, 98%, 99% or more) complementary to the transcription product of the target gene. To effectively inhibit expression of the target gene, the length of the antisense DNA is at least 15 bases or more, preferably 100 bases or more, and further preferably 500 bases or more. In general, the length of the antisense DNA to be used is shorter than 5 kb, and preferably shorter than 2.5 kb.

Meanwhile, in the method using the ribozyme technology, a DNA encoding an RNA having a ribozyme activity of specifically cleaving the transcription product of the target gene is used. Some ribozymes, such as those of the group I intron type, and M1RNA contained in RNaseP, have the sizes of 400 nucleotides or more, and others, called the hammerhead-type ribozymes or the hairpin-type ribozymes, have active domains of about 40 nucleotides (Makoto Koizumi and Eiko Ohtsuka, Tanpakushitsu Kakusan Kohso (Protein, Nucleic Acid, and Enzyme), 35: 2191, 1990).

For example, the self cleavage domain of a hammerhead-type ribozyme cleaves the 3' side of C 15 of G13U14C15. Formation of a base pair between U14 and A at position 9 is considered important for the activity, and it is shown that the cleavage occurs also when the base at position 15 is A or U, instead of C (Koizumi et. al., FEBS Lett. 228: 225, 1988). When a substrate binding site of the ribozyme is designed to be complementary to a RNA sequence near the target site, it is possible to create a restriction enzyme-like RNA cleaving ribozyme which recognizes a sequence of UC, UU, or UA in the target RNA (Koizumi et. al., FEBS Lett. 239: 285, 1988, Makoto Koizumi and Eiko Ohtsuka, Tanpakushitsu Kakusan Kohso (Protein, Nucleic Acid, and Enzyme), 35: 2191, 1990, Koizumi et. al., Nucleic. Acids. Res. 17: 7059, 1989).

The hairpin-type ribozymes are also useful for the present invention. A hairpin-type ribozyme is found, for example, in the minus strand of the satellite RNA of tobacco ringspot virus (Buzayan, Nature 323: 349, 1986). It is shown that the ribozyme can also be designed to cause target-specific RNA cleavage (Kikuchi and Sasaki, Nucleic Acids Res. 19: 6751, 1992, Kikuchi Yo, Kagaku To Seibutsu (Chemistry and Biology), 30: 112, 1992). The ribozyme designed to cleave the target is linked to a promoter such as the cauliflower mosaic virus 35S promoter and to a transcription termination sequence, so that the ribozyme can be transcribed in a plant cell. It is also possible to enhance the effect by arranging such structural units in tandem, so that cleavage can be caused at multiple sites in the target gene (Yuyama et al., Biochem. Biophys. Res. Commun. 186: 1271, 1992). By using such a ribozyme, it is possible to specifically cleave the transcription product of the target gene, and suppress the expression of the gene.

The vector for expressing a molecule which suppresses the function of the target gene in a plant cell and a method for introducing the vector into a plant cell are the same as the case of a vector for expressing a restriction endonuclease to be described later.

Meanwhile, in the method of disrupting a target gene, for example, a knockout technology utilizing homologous recombination can be used. In the knockout technology, a targeting DNA construct having a sequence homologous to a sequence of at least part of a target gene region is introduced into a cell, so that homologous recombination can occur with the target gene region. The targeting DNA construct typically has a structure in which DNAs comprising a sequence homologous to the sequence of the target DNA site abut on both ends of a DNA for disrupting the target gene. Specifically, the homologous DNAs are present in left and right arms of the targeting DNA construct, and the DNA for disrupting the target gene is positioned between the two arms. Here, the term "homologous" includes the cases where the sequences are partially different, in addition to the case where the sequences are completely (in other words, 100%) identical, as long as the homologous recombination occurs. Generally, the sequences are identical at least 95% or more, preferably 97% or more, further preferably 99% or more. The homologous sequences of the target gene region on the chromosome and the targeting DNA construct interact with each other, so that a specific sequence of the target gene region is exchanged with the DNA on the targeting DNA construct. As a result, the target gene can be knocked out. For preparation of the targeting DNA construct, for example, a DNA obtained by inserting a marker gene into a cloned target gene can be used.

In addition to these, it is also possible to use plant cells in which the target gene is disrupted by transposition of transposon, and plant cells in which the target gene is chemically disrupted by irradiation with high-energy electromagnetic waves such as ultraviolet rays, a mutagenic chemical, or the like, in the present invention.

Note that, for basic operations and methods of DNA recombination, construction of the vector, introduction of the vector into the cell, and the like used in the present invention, see a document (Sambrook et al., Molecular Cloning: A Laboratory Manual, Cold Spring Harbor, NY).

A plant from which the plant cell used in the present invention is derived is not particularly limited, and examples thereof include Arabidopsis, rice, barley, maize, tomato, soybean, potato, tobacco, and the like. The plant cell used in the present invention encompasses cells in plants, in addition to cultured cells. In addition, the plant cell used in the present invention also encompasses plant cells in various forms such as suspended cultured cells, protoplasts, leaf slices, calluses, immature embryos, and pollen.

In the present invention, a restriction endonuclease is expressed in the above-described plant cell, and double-strand break is induced at a restriction endonuclease target DNA site on a chromosome of the plant cell.

As the restriction endonuclease used in the present invention, a desired restriction endonuclease which has a recognition sequence at a target DNA site can be used. Here, the "target DNA site" means a position on a chromosome at which a mutation is intended to be introduced through nonhomologous end joining after the double-strand break.

A preferred restriction endonuclease used in the present invention is a zinc finger nuclease prepared by fusing a zinc finger domain which recognizes a specific nucleotidesequencewithanon-specificDNAcleavagedomain obtained from a restriction enzyme (chimeric restriction endonuclease) (International Publication No. WO00/46386, and International Publication No. WO03/080809). In preparation of the chimeric restriction endonuclease, the zinc finger domain and the non-specific DNA cleavage domain may be prepared as a single continuous unit, or may be produced separately and then linked to each other.

Moreover, a meganuclease enzyme which produces break on a chromosome at a low frequency can also be used in the present invention, as long as a recognition site thereof exists on the chromosome. The meganuclease enzyme is, for example, I-SceI which recognizes an 18-base sequence (5'-TAGGGATAA_{↓}CAGGGTAAT-3'), and which produces a 4-base 3'-OH protruding end. In the present invention, other various meganuclease enzymes such as I-ChuI, I-DmoI, I-CreI, I-CsmI, PI-SceI, and PI-PfuI can be used depending on a purpose. Moreover, a custom made meganuclease mutated to recognize and break a site other than that of an original meganuclease can also be used in the present invention depending on a purpose (International Publication No. W02004/067736).

For generating a double-strand break, the restriction endonuclease can be introduced into a plant cell as a vector comprising a nucleic acid encoding the restriction endonuclease, and then expressed therein. The vector for expression of the restriction endonuclease in the cell is not particularly limited, and various vectors can be used depending on a purpose. In the vector, the nucleic acid encoding the restriction endonuclease is functionally (in other words, in a state capable of being expressed in a cell) linked to at least one expression control sequence. The expression control sequence encompasses promoter sequences and enhancers. Examples of the promoter of the present invention for inducible expression of the DNA include promoters known to initiate expression in response to external factors such as high temperature, low temperature, dryness, ultraviolet ray irradiation, infection with or invasion of filamentous·fungi·bacteria·viruses, and spaying specific compounds. Examples of such promoters include the promoter of the Arabidopsis HSP18.2 gene and the promoters of the rice hsp80 and hsp72 genes, which are induced by high-temperature, the promoter of the rice lip19 gene, which is induced by low-temperature, the promoter of the Arabidopsis rab16 gene, which is induced by dryness, the promoter of the parsley chalcone synthase gene, which is induced by ultraviolet ray irradiation, the promoter of the maize alcohol dehydrogenase gene, which is induced under anaerobic conditions, the promoter of the rice chitinase gene and the promoter of the tobacco PR protein gene, which are expressed by infection with or invasion of filamentous fungi·bacteria·viruses, and the like. The rice chitinase gene promoter and the tobacco PR protein gene promoter are also induced by specific compounds such as salicylic acid, and the rab16 is also induced by spraying of a plant hormone, abscisic acid. Meanwhile, examples of the promoter for constitutive expression include the cauliflower mosaic virus 35S promoter, the rice actin promoter, the maize ubiquitin promoter, and the like.

For introduction of the vector into a plant cell, it is possible to use various methods known to whose skilled in the art such as the polyethylene glycol method, electroporation, the Agrobacterium-mediated method, the particle gun method.

In the plant cell, the target DNA site broken by the action of the restriction endonuclease is subjected to a repairing process by nonhomologous end joining. In the repairing process, a mutation may occur at the target DNA site. The plant cell produced by the method of the present invention is preferably one having the above-described non-silent mutation at the target DNA site.

A plant can be obtained by regenerating the plant cell produced by the method of the present invention. The function of the endogenous gene is suppressed in the plant cell into which the non-silent mutation is introduced at the target DNA site. Hence, the phenotype of the plant regenerated from such a plant cell may be changed, in association with the suppression of the function of the endogenous gene. Accordingly, the use of the method of the present invention makes it possible to efficiently perform breeding of a plant.

The regeneration of a plant from the plant cell can be carried out by a method known to those skilled in the art which depends on the kind of the plant cell. Examples thereof include the method described in Akama et al., (Plant Cell Reports 12: 7-11, 1992) for Arabidopsis; the method described in Datta (In Gene Transfer To Plants (Potrykus I and Spangenberg Eds.) pp 66-74, 1995), the method described in Toki et el, (Plant Physiol. 100: 1503-1507, 1992), the method described in Christou et al., (Bio/technology, 9: 957-962, 1991), and the method described in Hiei et al., (Plant J. 6: 271-282, 1994) for rice; the method described in Tingay et al., (Plant J. 11 : 1369-1376, 1997), the method described in Murray et al., (Plant Cell Report 22: 397-402, 2004), and the method described in Travalla et al., (Plant Cell Report 23: 780-789, 2005) for barley; the method described in Shillito et al., (Bio/Technology, 7: 581, 1989) and the method described in Gorden-Kamm et al., (Plant Cell 2: 603, 1990) for maize; the method described in Matsukura et al., (J. Exp. Bot., 44: 1837-1845, 1993) for tomato; the method described in a patent publication (United States (US) Patent No. 5,416,011) for soybean; the method described in Visser et al., (Theor. Appl. Genet, 78: 594, 1989) for potato; and the method described in Nagata and Takebe (Planta, 99: 12, 1971) for tobacco.

Once a transgenic plant in which a mutation is introduced into a target DNA on a chromosome is obtained, a progeny can be obtained from the plant by sexual reproduction or asexual reproduction. Moreover, it is also possible to obtain a propagation material (for example, seeds, fruits, cuttings, stubbles, calluses, protoplasts, or the like) from the plant or a progeny or clone of the plant, and then mass produce the plant from the propagation material. The present invention encompasses a plant cell in which a mutation is introduced into a target DNA on a chromosome, a plant comprising the cell, a progeny and a clone of the plant, and propagation materials of the plant, or a progeny or a clone of the plant.

The present invention also provides a kit for use in the above-described method of the present invention. The kit of the present invention comprises at least one authentic sample of any one of:
(a) a plant cell in which a function of a protein involved in repair of a broken double-stranded DNA is artificially suppressed;
(b) a DNA construct for artificially suppressing a function of a protein involved in repair of a broken double-stranded DNA in a plant cell (for example, a vector expressibly carrying the above-described DNA encoding a double-stranded RNA, the above-described DNA encoding an antisense RNA, or the above-described DNA encoding an RNA having a ribozyme activity); and
(c) a DNA construct for expressing a restriction endonuclease in a plant cell (for example, a vector expressibly carrying the above-described DNA encoding a restriction endonuclease). The kit of the present invention may further comprise an operations manual of the kit.

### [Example]

Hereinafter, the present invention will be described more specifically on the basis of Example. However, the present invention is not limited to Example below.

### (Example 1)

Introduction of mutations into Arabidopsis ABI4 gene using zinc finger nucleases, and effect of Ku80 deficiency on introduced mutations

To demonstrate zinc finger-mediated site-specific mutagenesis in Arabidopsis, the present inventors selected the ABI4 gene. The present inventors found full consensus target sites [5'-NNCNNCNNCNNNNNGNNGNNGNN-3' (N= A, C, G and T)/SEQ ID NO: 5] of zinc finger nucleases in ABI4.

In this Example, the method reported by Wright et al., (Wright DA, et al. Nat Protoc 1: 1637-1652, 2006) was used with slight modifications to assemble modules for constructing zincfinger nucleases. Amino acid sequences near specificity-determining residues are finger 1 "QRAHLER/SEQ ID NO: 6", finger 2 "QSGHLQR/SEQ ID NO: 7", and finger 3 "QRAHLER/SEQ ID NO: 8" in ZF-AAA (targeted for GGA GGA GGA) ; and finger 1 "RSDALTR/SEQ ID NO: 9", finger 2 "RSDDLQR/SEQ ID NO: 10", and finger 3 "RSDDLQR/SEQ ID NO: 11" in ZF-TCC (targeted for GTG GCG GCG). These coding sequences (annealed oligo DNAs shown in Table 1) of the zinc finger proteins were cloned between the XbaI and BamHI sites of the pGB-FB vector (Wright DA, et al. Nat Protoc 1: 1637-1652, 2006) (Fig. 1).

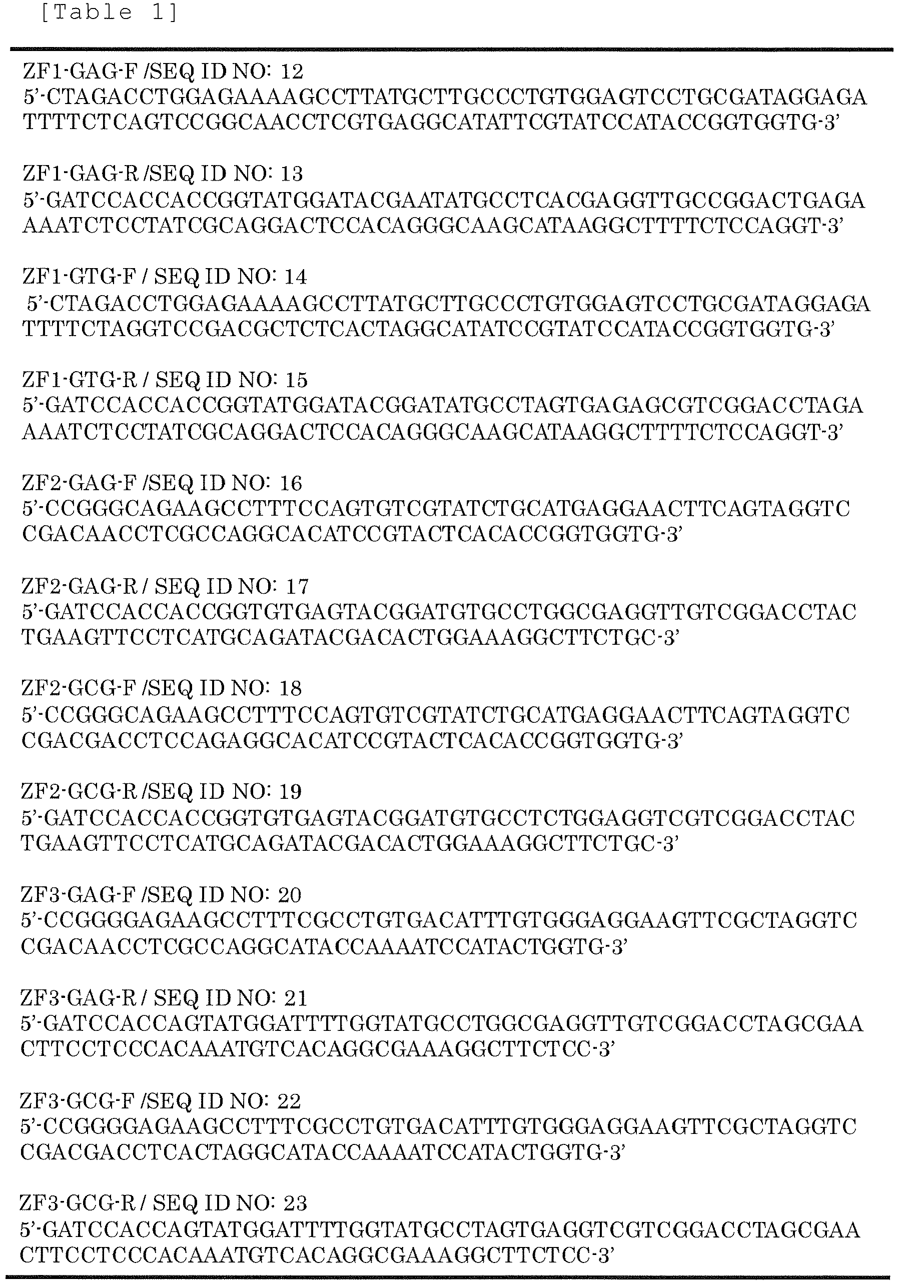

ZF1/ZF2/ZF3 domains were excised from the vector by XbaI-BamHI digestion, and inserted into a pP1.2gfPhsZFN vector to construct a zinc finger expression vector "pP1.2gfbPhsZFN-ABI4" (Fig. 2). This plasmid has a promoter of the Arabidopsis heat shock protein HSP18.2 gene (Takahashi T, et al., Plant J 2: 751-761, 1992), and is capable of transiently expressing zinc finger nucleases upon heat shock. For this reason, this plasmid has the benefit of avoiding cytotoxicity (Porteus MH, Mol Ther 13 : 438-446, 2006).

To determine whether or not the induction of a zinc finger nuclease activity results in digestion of the Arabidopsis genome in vivo so that mutations can be induced at recognition sequences in Arabidopsis cells, the vector was introduced into an Agrobacterium tumefaciens strain GV3101 by electroporation, and then introduced into Arabidopsis by the floral dipping method (Clough SJ & Bent AF, Plant J 16: 735-743, 1998). Transgenic plants were selected on culture medium containing 1.5 µg/ml blasticidin-S on the basis of the appearance of GFP fluorescence (Ochiai-Fukuda T, et al. J Biotechnol 122: 521-527, 2005). Seedlings were grown on plates at 22°C for 12 days. Then, the plates were wrapped with plastic wrap, and immersed in water at 40°C for 90 minutes to apply heat shock. The seedlings were further grown at 22°C for 24 hours, and then DNA was extracted. The present inventors extracted DNA from true leaves of these lines before the heat induction, for non-heat induced control experiment.

In order to determine whether or not induction of a zinc finger activity can induce mutations in recognition sequence, the present inventors have developed a method using a mismatch-specific endonuclease. In this method, SURVEYOR Mutation Detection Kit (Transgenomic) was used to detect mutations induced with the zinc finger nucleases in the ABI4 gene. A 685-base pair region surrounding a ZFN-AAA/ZFN-TCC pair portion was PCR-amplified by use of a high-fidelity DNA polymerase KOD-Plus (TOYOBO). PCR reactions (50 µl) were carried out by use of a 2.5 units of KOD-Plus (TOYOBO) in a buffer containing 0.2 mM dNTPs and 1 mM primers (ABI4-F1: 5'-TGGACCCTTTAGCTTCCCAACATCAACACA-3'/SEQ ID NO: 24, and ABI4-R1: 5'-ACCGGAACATCAGTGAGCTCGATGTCAGAA-3'/SEQ ID NO: 25). Approximately 30 ng of genomic DNA was used as a template in each PCR reaction. PCR reaction program was as follows: denaturation at 95°C for 15 seconds, and subsequent 35 cycles of "95°C for 15 seconds, 65°C for 15 seconds, 68 °C for 60 seconds; and final extension at 68 °C for 7 minutes." To determine zinc finger nuclease-induced mutations, initial PCR products were cloned into the pCR-TOPO vector (Invitrogen), and single colonies each containing an ABI4 gene fragment were prepared. These colonies were subjected to PCR reactions again, and the obtained products were analyzed by the above-described SURVEYOR nuclease assay. Then, a plasmid was isolated from a single colony showing mutation in this analysis, and the base sequence thereof was determined.

As a result of the analysis, no clones containing a mutation were found in control seedlings, in which the zinc finger nuclease was not induced by heat shock. Meanwhile, mutations were observed in target sequences of lines to which heat shock was applied. However, most of the mutations were of a substitution type which did not involve large deletions or insertions.

The present inventors hypothesized that an activity of the Ku protein inhibited drastic modifications such as large deletions and/or insertions at DNA double-strand break ends. To test this hypothesis, the present inventors investigated the type and frequency of mutations in the nonhomologous end joining-deficient Arabidopsis mutant, "atku80" (West SC, et al., Plant J 31: 517-528, 2002). Zinc finger nucleases were introduced into protoplasts prepared by pP1.2gfbPhsZFN-ABI4 from wild-type and atku80 plants, by the PEG-calcium method (Yoo S-D, et al., Nat protocols 2: 1565-1572, 2007), and expressed transiently. Then, mutations were analyzed as described above.

Fig. 3 shows the frequencies and distributions of mutations of the wild-type plant and the atku80 plant. In atku80 cells, the percentage of occurrences of repair which was expressed in the phenotype was increased 2.7 times (Figs. 3A and B). Especially, deletions of 4 bases or more were drastically increased, and were 20 times more frequent than those in the wild type (Fig. 3 A). However, the frequency of mutations in the atku80 cells was the same as that of the wild type (Fig. 3 C). Overall, the present inventors have concluded that AtKu80 functions to avoid end degradation with zinc finger nucleases at DNA double-strand break sites. However, since the frequency of mutations was unchanged, it is conceivable that the frequency of repair with high fidelity by homologous recombination after cutting of the DNA double-strand break ends is increased in the atku80 mutant.

### [Industrial Applicability]

The present invention makes it possible to dramatically improve the frequency of introductions of non-silent mutations into target DNA sites in production of genetically modified plant cells through a repairing process by nonhomologous end joining after DNA double-strand break with a restriction endonuclease. The efficiency of introductions of non-silent mutations by DNA double-strand break has so far been low, especially in higher plants, and the low efficiency has been an obstacle to putting the introductions of non-silent mutations by DNA double-strand break into practical use. However, the present invention makes it possible to dramatically increase the efficiency of the introductions. Accordingly, the present invention will greatly contribute to not only functional analysis of plant genes, but also development of selective breeding of plants.

### [Sequence Listing Free Text]

SEQ ID NO: 5
   <223> n represents a, c, g, or t.
   <223> Consensus target sites of ZFNs
SEQ ID NOs: 6 to 11
   <223>Amino acid sequences of artificially synthesized zinc fingers
SEQ ID NOs: 12 to 23
   <223> Base sequences encoding artificially synthesized zinc fingers
SEQ ID NOs: 24 and 25
   <223> Base sequences of artificially synthesized primers

## Claims

1. A method for producing a genetically modified plant cell, comprising:
expressing a restriction endonuclease in a plant cell in which a function of a protein involved in repair by nonhomologous end joining is artificially suppressed, thereby inducing a double-strand break at a target DNA site of the restriction endonuclease on a chromosome of the plant cell; and
causing a mutation at the target DNA site through a repairing process by nonhomologous end joining at the broken target DNA site of the plant cell.

2. The method according to claim 1, wherein the protein involved in the repair by the nonhomologous end joining is at least one of Ku70 and Ku80.

3. The method according to claim 1 or 2, wherein the restriction endonuclease is a zinc finger nuclease.

4. A genetically modified plant cell which is produced by the method according to any one of claims 1 to 3.

5. A plant comprising the cell according to claim 4.

6. A plant which is a progeny or a clone of the plant according to claim 5.

7. A propagation material of the plant according to claim 5 or 6.

8. A kit for use in the method according to any one of claims 1 to 3, comprising at least one of the following (a) to (c):
(a) a plant cell in which a function of a protein involved in repair of a broken double-stranded DNA is artificially suppressed;
(b) a DNA construct for artificially suppressing a function of a protein involved in repair of a broken double-stranded DNA in a plant cell; and
(c) a DNA construct for expressing a restriction endonuclease in a plant cell.
